(19) **Europäisches Patentamt / European Patent Office / Office européen des brevets**

(11) **EP 4 520 259 A1**

(12) **EUROPEAN PATENT APPLICATION**
published in accordance with Art. 153(4) EPC

(43) Date of publication:
**12.03.2025 Bulletin 2025/11**

(21) Application number: **23799427.2**

(22) Date of filing: **10.04.2023**

(51) International Patent Classification (IPC):
$A61B\ 5/02^{(2006.01)}$     $A61B\ 5/00^{(2006.01)}$
$A61B\ 5/01^{(2006.01)}$     $A61B\ 5/021^{(2006.01)}$
$A61B\ 5/0245^{(2006.01)}$   $A61B\ 5/08^{(2006.01)}$
$A61B\ 5/1171^{(2016.01)}$   $A61B\ 5/1455^{(2006.01)}$
$G02B\ 27/02^{(2006.01)}$    $H04N\ 5/64^{(2006.01)}$

(52) Cooperative Patent Classification (CPC):
A61B 5/00; A61B 5/01; A61B 5/02; A61B 5/021;
A61B 5/0245; A61B 5/08; A61B 5/1171;
A61B 5/1455; G02B 27/02; H04N 5/64

(86) International application number:
**PCT/JP2023/014497**

(87) International publication number:
**WO 2023/214495 (09.11.2023 Gazette 2023/45)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **06.05.2022 JP 2022076466**

(71) Applicant: **Sony Group Corporation
Tokyo 108-0075 (JP)**

(72) Inventor: **NISHIMURA, Kiminobu
Tokyo 108-0075 (JP)**

(74) Representative: **MFG Patentanwälte
Meyer-Wildhagen Meggle-Freund
Gerhard PartG mbB
Amalienstraße 62
80799 München (DE)**

(54) **BIOLOGICAL INFORMATION MEASUREMENT DEVICE, BIOLOGICAL INFORMATION MEASUREMENT METHOD, AND BIOLOGICAL INFORMATION MEASUREMENT PROGRAM**

(57)     [Object] To provide a biological information measuring apparatus, a biological information measuring method, and a biological information measuring program that make it possible to measure biological information while reducing burdens imposed on a user.

[Solving Means] A biological information measuring apparatus according to the present technology includes a reflection spectrum generator and an analysis processor. The reflection spectrum generator generates a reflection spectrum that is a wavelength spectrum of reflected light obtained by irradiation light irradiated onto a measurement area being reflected off the measurement area, the irradiation light making up a display video, the measurement area being a specific area on a body of a user. The analysis processor calculates biological information regarding the user on the basis of an irradiation spectrum and the reflection spectrum, the irradiation spectrum being a wavelength spectrum of the irradiation light.

FIG.1

## Description

Technical Field

The present technology relates to a biological information measuring apparatus, a biological information measuring method, and a biological information measuring program that are used to measure biological information regarding a user using light.

Background Art

Measurement of biological information such as a pulse rate, arterial oxygen saturation, and a body-surface temperature is called biological sensing. Favorably, the biological sensing not imposing heavy burdens on users is used. Particularly when, for example, biological information is measured for a long time at home, the level of burden could have a great impact on the measurement of biological information. In this regard, biological sensing using light does not impose heavy burdens on users, but is easily affected by external light. Thus, there is a need to take measures against this matter.

For example, Patent Literature 1 discloses an apparatus that is included in a head-mounted display. The apparatus causes a display panel to emit light on a specified rule, images reflected light reflected off the skin or the eyeball of a user, and checks image information against registered information. Accordingly, the apparatus performs individual identification on the user. The use of a head-mounted display makes it possible to block external light, and thus to perform individual identification with a high degree of accuracy.

Citation List

Patent Literature

Patent Literature 1: Japanese Patent Application Laid-open No. 2021-18729

Disclosure of Invention

Technical Problem

However, when such an approach disclosed in Patent Literature 1 is applied, there is a need to cause a display panel to emit light on a specified rule. The emission of light on a specified rule includes causing a display panel to emit light of a specific color and performing switching of the color in a sequential manner; and causing pieces of light of different colors to be emitted for respective divisions of the display panel. The light does not have particular significance for a user, and it is necessary for the user to wait until identification processing is completed. Thus, there are still burdens imposed on a user.

In view of the circumstances described above, it is an object of the present technology to provide a biological information measuring apparatus, a biological information measuring method, and a biological information measuring program that make it possible to measure biological information while reducing burdens imposed on a user.

Solution to Problem

In order to achieve the object described above, a biological information measuring apparatus according to an embodiment of the present technology includes a reflection spectrum generator and an analysis processor.

The reflection spectrum generator generates a reflection spectrum that is a wavelength spectrum of reflected light obtained by irradiation light irradiated onto a measurement area being reflected off the measurement area, the irradiation light making up a display video, the measurement area being a specific area on a body of a user.

The analysis processor calculates biological information regarding the user on the basis of an irradiation spectrum and the reflection spectrum, the irradiation spectrum being a wavelength spectrum of the irradiation light.

The reflection spectrum generator may generate the reflection spectrum from reflected-light-reception data that is data generated by the reflected light being imaged.

The biological information measuring apparatus may further include an irradiation spectrum calculator that calculates the irradiation spectrum on the basis of video data of the display video and on the basis of device characteristics of the biological information measuring apparatus, and the spectrum acquisition section may acquire the irradiation spectrum from the irradiation spectrum calculator.

The biological information measuring apparatus may further include an irradiation spectrum generator that generates the irradiation spectrum from irradiation-light-reception data that is data generated by the irradiation light being imaged, and the spectrum acquisition section may acquire the irradiation spectrum from the irradiation spectrum calculator.

The biological information measuring apparatus may further include a display section that displays thereon the display video, and a reflected light imaging section that images the reflected light to generate reflected-light-reception data.

The biological information measuring apparatus may further include an irradiation light imaging section that images the irradiation light to generate irradiation-light-reception data.

The biological information measuring apparatus may be a head-mounted display that is worn on a head of the user and of which the display section is arranged in front of eyes of the user.

The reflected light imaging section may be arranged to face the measurement area.

The biological information measuring apparatus may further include an irradiation light imaging section that images the irradiation light to generate irradiation-light-reception data, and the irradiation light imaging section may be arranged to face the display section.

The display section and the reflected light imaging section may each be spaced from the user, and the biological information measuring apparatus may further include a measurement area recognizing section that recognizes the measurement area on the basis of an image obtained by imaging being performed by the reflected light imaging section.

The analysis processor may determine the biological information regarding the user using a biological information calculating model obtained by performing modeling on the irradiation spectrum, the reflection spectrum, and the biological information for each type of biological information.

The biological information calculating model may be a predictive model that is generated by machine learning.

The biological information may be a pulse rate, arterial oxygen saturation, a skin-moisture amount, a body-surface temperature, a breathing rate, or a blood pressure.

The biological information may be a vein pattern or an iris pattern, and the analysis processor may perform biometric authentication using the biological information.

In order to achieve the object described above, a biological information measuring program according to an embodiment of the present technology causes an information processing apparatus to operate as a reflection spectrum generator and an analysis processor.

The reflection spectrum generator generates a reflection spectrum that is a wavelength spectrum of reflected light obtained by irradiation light irradiated onto a measurement area being reflected off the measurement area, the irradiation light making up a display video, the measurement area being a specific area on a body of a user.

The analysis processor calculates biological information regarding the user on the basis of an irradiation spectrum and the reflection spectrum, the irradiation spectrum being a wavelength spectrum of the irradiation light.

In order to achieve the object described above, a biological information measuring method according to an embodiment of the present technology includes generating a reflection spectrum that is a wavelength spectrum of reflected light obtained by irradiation light irradiated onto a measurement area being reflected off the measurement area, the irradiation light making up a display video, the measurement area being a specific area on a body of a user.

Biological information regarding the user is calculated on the basis of an irradiation spectrum and the reflection spectrum, the irradiation spectrum being a wavelength spectrum of the irradiation light.

Brief Description of Drawings

[Fig. 1] Fig. 1 schematically illustrates a biological information detecting apparatus according to a first embodiment of the present technology.

[Fig. 2] Fig. 2 schematically illustrates a measurement area.

[Fig. 3] Fig. 3 is a block diagram illustrating a functional configuration of the biological information detecting apparatus.

[Fig. 4] Fig. 4 illustrates an example of an irradiation spectrum calculated by an irradiation spectrum calculator included in the biological information detecting apparatus.

[Fig. 5] Fig. 5 illustrates an example of a reflection spectrum calculated by a reflection spectrum generator included in the biological information detecting apparatus.

[Fig. 6] Fig. 6 is a flowchart illustrating an operation of the biological information detecting apparatus.

[Fig. 7] Fig. 7 schematically illustrates how to deal with an offset of the biological information detecting apparatus.

[Fig. 8] Fig. 8 schematically illustrates how to deal with the offset of the biological information detecting apparatus.

[Fig. 9] Fig. 9 schematically illustrates the biological information detecting apparatus having another configuration.

[Fig. 10] Fig. 10 is a block diagram illustrating a functional configuration of the biological information detecting apparatus having the other configuration.

[Fig. 11] Fig. 11 schematically illustrates a biological information detecting apparatus according to a second embodiment of the present technology.

[Fig. 12] Fig. 12 is a block diagram illustrating a functional configuration of the biological information detecting apparatus.

[Fig. 13] Fig. 13 is a flowchart illustrating an operation of the biological information detecting apparatus.

[Fig. 14] Fig. 14 is a block diagram illustrating a hardware configuration of each of the biological information detecting apparatus according to the first embodiment of the present technology and the biological information detecting apparatus according to the second embodiment of the present technology.

Mode(s) for Carrying Out the Invention

(First Embodiment)

A biological information detecting apparatus according to a first embodiment of the present technology is described.

[Configuration of Biological Information Detecting Apparatus]

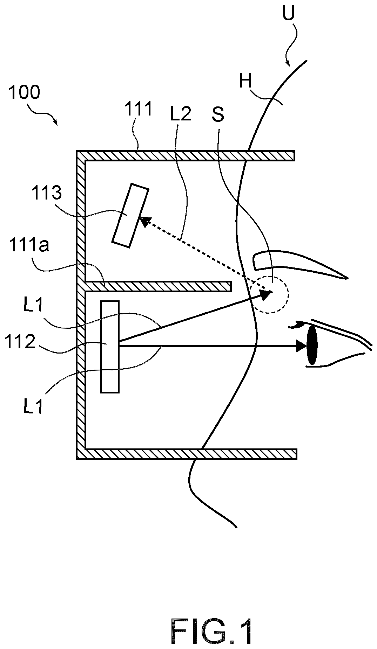

Fig. 1 schematically illustrates a biological information measuring apparatus 100 according to the present embodiment. As illustrated in the figure, the biological information measuring apparatus 100 is a head-mounted display worn on a head H of a user U, and includes a housing 111, a display section 112, and a reflected light imaging section 113.

The housing 111 is worn on the head H, and supports the display section 112 and the reflected light imaging section 113. A partition 111a that separates the display section 112 from the reflected light imaging section 113 is provided to the housing 111. A specific configuration of the housing 111 is not particularly limited. It is sufficient if the housing 111 can maintain positions of the display section 112 and the reflected light imaging section 113 relative to the head H, and, favorably, the housing 111 excellently blocks external light.

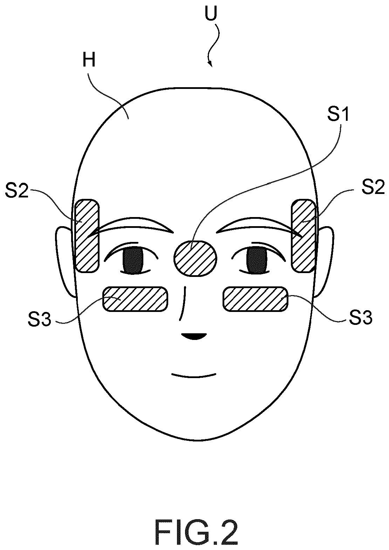

The display section 112 is arranged in front of eyes of the user U, and displays thereon a video. The video displayed on the display section 112 is hereinafter referred to as a "display video", and light that makes up the display video is hereinafter referred to as "irradiation light". Fig. 1 illustrates irradiation light L1 as the irradiation light. The irradiation light L1 is light incident on a facial surface of the user U, and includes emitted light emitted from the display section 112, and light obtained by the emitted light being reflected inside of the housing 111. As illustrated in Fig. 2, the user U can view a display video by use of the irradiation light L1 incident on the eyes of the user U. Further, a portion of the irradiation light L1 is reflected off the facial surface of the user U to become reflected light. Fig. 1 illustrates reflected light L2 as the reflected light.

A device from which a light amount of emitted light is larger than a specified light amount can be used as the display section 112, and, for example, a liquid crystal display can be used as the display section 112. A single display section 112 may be provided, or two display sections 112 for a left eye and for a right eye may be provided. Note that there is a head-mounted display to which another display apparatus such as a smartphone is attachable, and the other display apparatus can also be used as the display section 112. Further, an optical element such as a lens may be arranged between the display section 112 and the eyes of the user U.

The reflected light imaging section 113 images the reflected light L2 to generate reflected-light-reception data that is data of reception of the reflected light L2. The reflected light imaging section 113 performs imaging on a "measurement area" on the facial surface of the user U. The measurement area refers to a specific area on the facial surface of the user U. Fig. 1 illustrates a measurement area S as the measurement area. Fig. 2 schematically illustrates an example of the measurement area S. The figure illustrates an area S1 that corresponds to an area between eyes, an area S2 that corresponds to a temple, and an area S3 that corresponds to an area under the eye. The measurement area S may include at least one of these areas. Further, the measurement area S may be an area, on the facial surface of the user U, that is other than the areas S1 to S3. The reflected light imaging section 113 faces the measurement area S, and images the reflected light L2 reflected off the measurement area S. Note that an optical element such as a lens may be arranged between the reflected light imaging section 113 and the facial surface of the user U.

The reflected light imaging section 113 may be a general imaging device, or may be an imaging device that is included in a multispectral camera (MSC). A general camera performs image-capturing on three channels of red, green, and blue using light-receiving elements provided with respective color filters of red, green, and blue. On the other hand, the MSC is a camera that can perform image-capturing on a larger number of channels such as nine channels.

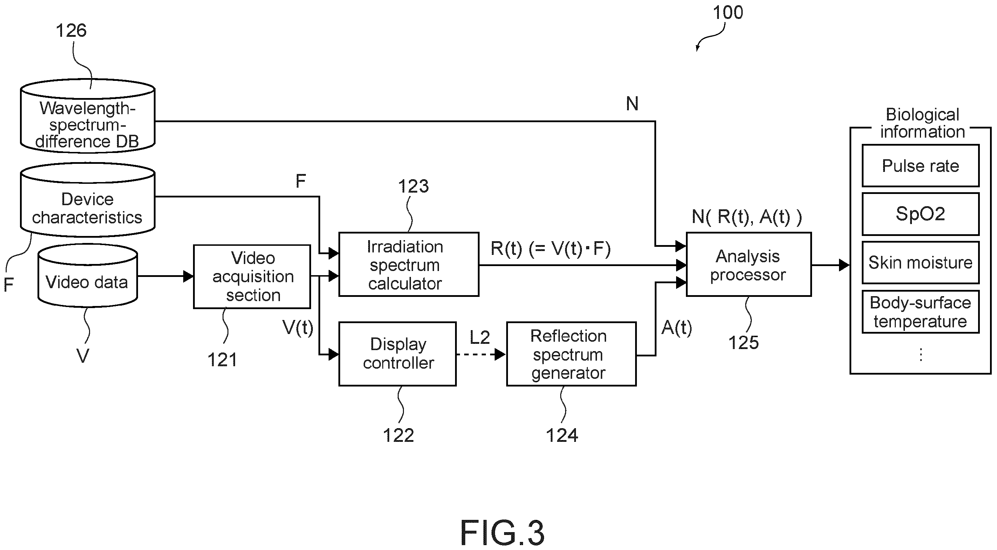

Fig. 3 is a block diagram illustrating a functional configuration of the biological information measuring apparatus 100. As illustrated in the figure, the biological information measuring apparatus 100 includes a video acquisition section 121, a display controller 122, an irradiation spectrum calculator 123, a reflection spectrum generator 124, and an analysis processor 125. The functional configuration is implemented by software and hardware working cooperatively. The functional configuration may be included in the housing 111 or in an apparatus external to the housing 111.

The video acquisition section 121 acquires video data V, and generates video data V(t) making up a display video displayed on the display section 112. The video acquisition section 121 can acquire the video data V from, for example, a storage included in the biological information measuring apparatus 100, an external apparatus, or a network. Details of the video data V are not particularly limited, and the video data V may be a moving image such as a movie or a game video, or a still image. The video data V(t) is represented as below.

$$V(t) = [V_r(t); V_g(t); V_b(t)] \qquad \text{(Formula 1)}$$

In (Formula 1), $V_r(t)$ represents the intensity of a red component in a display video, $V_g(t)$ represents the intensity of a green component in the display video, and $V_b(t)$ represents the intensity of a blue component in the display video. The video acquisition section 121 supplies the generated video data V(t) to the display controller 122 and the irradiation spectrum calculator 123.

The display controller 122 controls the display section 112 (refer to Fig. 1) to cause video data V(t) supplied by the video acquisition section 121 to be displayed on the display section 112. Accordingly, the irradiation light L1 making up a display video is irradiated onto a facial surface of a user.

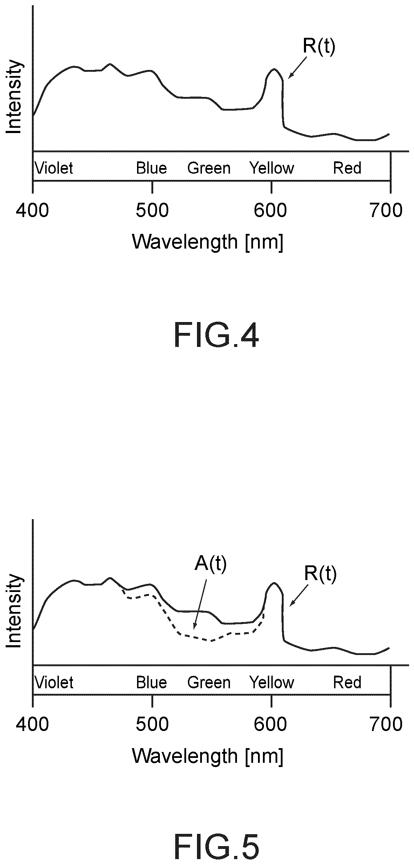

The irradiation spectrum calculator 123 calculates an irradiation spectrum on the basis of video data V(t) and device characteristics F of the biological information measuring apparatus 100. The irradiation spectrum is a wavelength spectrum of the irradiation light L1, and Fig. 4 illustrates an example of the irradiation spectrum.

The device characteristics F are characteristics that represent a relationship between emitted light emitted from the display section 112 and the irradiation light L1 incident on a facial surface of a user. The emitted light emitted from the display section 112 is affected by, for example, display characteristics of the display section 112, characteristics of an optical element, reflection on an inner peripheral surface of the housing 111, and a position of the measurement area S, and the device characteristics F include these effects. The device characteristics F can be obtained by performing measurement in advance. Specifically, an imaging device of which characteristics are known is arranged for the measurement area S, and light incident from the display section 112 is received by the imaging device. Accordingly, the device characteristics F can be obtained. The device characteristics F are represented as below.

$$F = [f_{r1}, f_{r2} \ \dots \ f_{rN};$$
$$f_{g1}, f_{g2} \ \dots \ f_{gN};$$
$$f_{b1}, f_{b2} \ \dots \ f_{bN}] \qquad \text{(Formula 2)}$$

In (Formula 2), N represents a division number (the number of bins) for a spectrum, and is, for example, 512. This means that a wavelength band of an irradiation spectrum is divided into 512 divisions to perform calculation. A light-receiving element with a red color filter when an imaging device used to measure the device characteristics F is arranged for the measurement area S is referred to as a red light-receiving element, a light-receiving element with a green color filter when the imaging device is arranged for the measurement area S is referred to as a green light-receiving element, and a light-receiving element with a blue color filter when the imaging device is arranged for the measurement area S is referred to as a blue light-receiving element. In (Formula 2), $f_{r1}$ represents a rate of change in the intensity of light received by the red light-receiving element in a first division, $f_{r2}$ represents a rate of change in the intensity of the light received by the red light-receiving element in a second division, and $f_{rN}$ represents a rate of change in the intensity of the light received by the red light-receiving element in an N-th division. The same applies to the green light-receiving element ($f_g$) and the blue light-receiving element ($f_b$).

It is assumed that the device characteristics F are dependent on the number of channels of the reflected light imaging section 113. In (Formula 2), the device characteristics F refer to display characteristics when the reflected light imaging section 113 includes three channels of red, green, and blue. It is assumed that the device characteristics F are dependent on the number of channels of an MSC when the reflected light imaging section 113 is the MSC.

The irradiation spectrum calculator 123 multiplies the video data V(t) by the device characteristics F. This makes it possible to calculate an irradiation spectrum. The irradiation spectrum calculated by the irradiation spectrum calculator 123 is hereinafter referred to as an irradiation spectrum R(t). The irradiation spectrum R(t) is represented as below.

$$R(t) = V(t)F = [r_1(t), r_2(t), \ \dots \ r_N(t)] \qquad \text{(Formula 3)}$$

In (Formula 3), $r_1$ represents the intensity in the first division, $r_2$ represents the intensity in the second division, and $r_N$ represents the intensity in the N-th division. As described above, the irradiation spectrum calculator 123 reflects, in emitted light emitted from the display section 112, the display characteristics of the display section 112 and reflection characteristics of the housing 111 to calculate a wavelength spectrum of the irradiation light L1, that is, an irradiation spectrum. Note that the irradiation spectrum calculator 123 averages pixels of video data V(t) to make the resolution lower.

This also makes it possible to reduce a calculation amount. The irradiation spectrum calculator 123 supplies the calculated irradiation spectrum R(t) to the analysis processor 125.

The reflection spectrum generator 124 acquires reflected-light-reception data from the reflected light imaging section 113, and generates a reflection spectrum A(t) from the reflected-light-reception data. The reflection spectrum A(t) is a wavelength spectrum of the reflected light L2, and is represented as below.

$$A(t) = [a_1(t), a_2(t), \dots a_M(t)] \quad \text{(Formula 4)}$$

In (Formula 4), $a_i$ represents an amount of light output by a light-receiving element of a channel i of the reflected light imaging section 113. Further, M represents the number of channels of the reflected light imaging section 113. The reflection spectrum generator 124 may average amounts of pieces of light respectively output by all of the light-receiving elements of the reflected light imaging section 113 to obtain a scalar, or may separately process amounts of pieces of light respectively output by the light-receiving elements. Further, the reflection spectrum generator 124 may average to process amounts of pieces of light respectively output by a plurality of light-receiving elements. When there are a large number of processing-target light-receiving elements, a biological information calculating model described later will get complicated. The reflection spectrum generator 124 supplies the generated reflection spectrum A(t) to the analysis processor 125.

The analysis processor 125 calculates biological information regarding a user on the basis of the irradiation spectrum R(t) and the reflection spectrum A(t). Fig. 5 is a graph in which examples of the irradiation spectrum R(t) and the reflection spectrum A(t) are given. As illustrated in the figure, there is a difference between the irradiation spectrum R(t) and the reflection spectrum A(t). Biological information in the measurement area S is reflected in characteristics of the difference, that is, in a wavelength band in which there is the difference and in an amount of change in intensity in which there is the difference. Thus, the analysis processor 125 can calculate biological information regarding a user on the basis of the irradiation spectrum R(t) and the reflection spectrum A(t).

Specifically, the analysis processor 125 can acquire a biological information calculating model N from the wavelength-spectrum-difference database 126. The biological information calculating model N is obtained by performing modeling on an irradiation spectrum R(t), a reflection spectrum A(t), and biological information for each type of biological information. As represented by (Formula 5) indicated below, the analysis processor 125 can calculate biological information (index) by applying the irradiation spectrum R(t) and the reflection spectrum A(t) to the biological information calculating model N.

$$N(R(t), A(t)) = \text{index} \quad \text{(Formula 5)}$$

Examples of the biological information that can be calculated using the biological information calculating model N include a pulse rate, arterial oxygen saturation (SpO2), a skin-moisture amount, a body-surface temperature, a breathing rate, a blood pressure, a vein pattern, and an iris pattern, and the biological information calculating model N is provided for each type of biological information. The analysis processor 125 selects the biological information calculating model N according to the type of biological information to be calculated, and can use the selected biological information calculating model N to calculate biological information.

The biological information calculating model N may be a predictive model generated by a machine learning approach in which the irradiation spectrum R(t) and the reflection spectrum A(t) are used as input and biological information measured using, for example, a wearable device is used as training data. Further, the biological information calculating model N is not limited to a model generated by machine learning. A specific example of biological information calculated by the analysis processor 125 using the biological information calculating model N is described below.

The analysis processor 125 may perform filtering using a green-wavelength-band filter, perform fast Fourier transform (FFT), perform filtering using a band pass filter (BPF) of between 1 Hz and 2.5 Hz, inclusive, and detect a peak with respect to each of the irradiation spectrum R(t) and the reflection spectrum A(t) to set a value of the peak to be a pulse rate. Further, the analysis processor 125 may perform filtering using a red-wavelength-band or near-infrared-wavelength-band filter with respect to each of the irradiation spectrum R(t) and the reflection spectrum A(t) to calculate $SpO_2$ on the basis of a ratio of respective results of the filtering.

Furthermore, the analysis processor 125 may calculate a body-surface temperature on the basis of the intensities of the irradiation spectrum R(t) and the reflection spectrum A(t) in a far-infrared wavelength band and on the basis of a value obtained by a temperature sensor. The reason is that a portion of the far-infrared wavelength is related to a temperature close to a body temperature. Further, the analysis processor 125 may perform filtering using a near-infrared-wavelength-band filter with respect to each of the irradiation spectrum R(t) and the reflection spectrum A(t) to calculate a skin-moisture amount on the basis of a ratio of respective results of the filtering. Veins absorb light of a near-infrared wavelength band, which is different from arteries. Thus, a wavelength band that is different from the green wavelength band used for the pulse rate is used.

Moreover, the analysis processor 125 may perform filtering using a green-wavelength-band filter, perform filtering using a BPF of between 1 Hz and 2.5 Hz, inclusive, and detect an envelope with respect to each of the irradiation spectrum R(t) and the reflection spectrum A(t) to calculate a breathing rate. Further, the analysis processor 125 may perform filtering using a green-wavelength-band filter, perform filtering using a BPF of between 1 Hz and 2.5 Hz, inclusive, and detect a peak with respect to each of different measurement areas S on a facial surface to calculate a blood pressure on the basis of a propagation speed at the peak. Waves of blood delivered from the heart can be observed as pulse waves. Thus, the pulse wave is measured at two points in the same path, and a speed at which the pulse wave propagates is obtained. Accordingly, a relative blood pressure can be estimated (cuffless blood-pressure estimation performed by a pulse-transit-time approach).

Furthermore, the analysis processor 125 may recognize a vein pattern of a face on the basis of two-dimensional information obtained by performing filtering using a green-wavelength-band filter with respect to each of the irradiation spectrum R(t) and the reflection spectrum A(t), and may perform biometric authentication on a user. Moreover, the analysis processor 125 may recognize an iris pattern of an eye on the basis of two-dimensional information obtained by performing filtering using an infrared-wavelength-band filter with respect to each of the irradiation spectrum R(t) and the reflection spectrum A(t), and may perform biometric authentication on a user.

The biological information calculated by the analysis processor 125 is not limited to the examples described above, and any biological information that can be calculated on the basis of the irradiation spectrum R(t) and the reflection spectrum A(t) may be adopted.

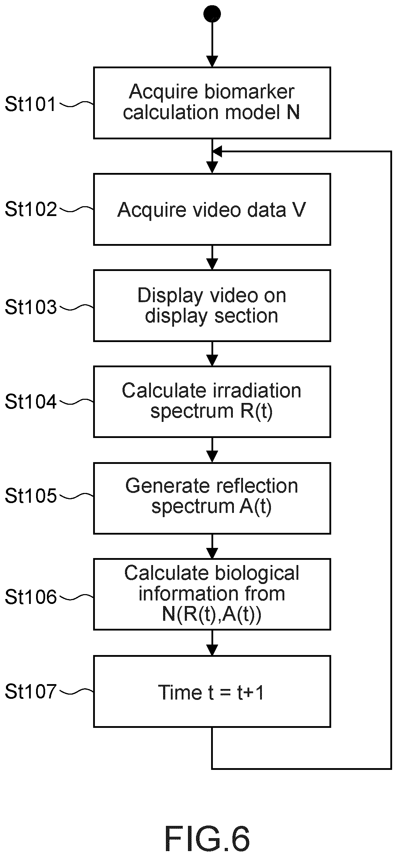

[Operation of Biological Information Detecting Apparatus] An operation of the biological information measuring apparatus 100 is described. Fig. 6 is a flowchart illustrating the operation of the biological information measuring apparatus 100.

When the biological information measuring apparatus 100 starts operating, the analysis processor 125 acquires a biological information calculation model N for calculation-target biological information from the wavelength-spectrum-difference database 126 (St101). Subsequently, the video acquisition section 121 acquires video data V (St102), and the display controller 122 supplies video data V(t) to the display section 112 to cause a display video to be displayed on the display section 112 (St103).

Subsequently, the irradiation spectrum calculator 123 calculates an irradiation spectrum R(t) on the basis of the video data V(t) and device characteristics F (St104). Subsequently, the reflection spectrum generator 124 acquires reflected-light-reception data from the reflected light imaging section 113, and generates a reflection spectrum A(t) from the reflected-light-reception data (St105).

Subsequently, the analysis processor 125 applies the irradiation spectrum R(t) and the reflection spectrum A(t) to the biological information calculating model N, and calculates biological information from N(R(t),A(t)) (St106). Subsequently, the analysis processor 125 changes the time from a time t to a time t+1 (St107), and repeatedly performs the processes of and after the acquisition of the biological information calculating model N (St101) again. Note that the time t corresponds to a frame of the video data V(t), and the time t+1 means addition of one frame.

The biological information measuring apparatus 100 measures biological information as described above. Note that the reflected-light-reception data may be stored upon the generation of the reflection spectrum A(t) (St105), and a processing apparatus such as a server with a high computational performance may calculate N(R(t),A(t)) using the reflected-light-reception data afterward together with the video data V(t) and the device characteristics F.

[Effects Provided by Biological Information Detecting Apparatus]

As described above, the biological information measuring apparatus 100 can measure biological information on the basis of a difference between light irradiated onto a facial surface of a user and light reflected off the facial surface of the user. The light irradiated onto the facial surface of the user is light making up a display video displayed on the display section 112, and details of the display video are not particularly limited. Thus, biological information can be measured while a user is viewing any video such as a movie or a game video. This results in a reduction in burdens imposed on the user upon measuring the biological information. Thus, the biological information measuring apparatus 100 is also suitable to measure biological information every day or for a long time at home.

[How to Deal With Offset of Head-Mounted Display]

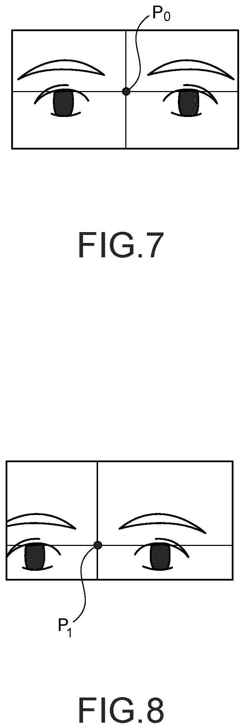

When a head-mounted display that is the biological information measuring apparatus 100 is offset relative to the head H of the user U, the biological information measuring apparatus 100 can deal with the offset as indicated below. Figs. 7 and 8 illustrate examples of captured images each obtained using the reflected light imaging section 113.

The analysis processor 125 detects approach of a face by use of, for example, a proximity sensor using infrared light, and sets the time of the detection to be an initial time $t_0$. With respect to amounts $A_{all}(t_0)$ of pieces of light received by all of the light-receiving elements of the reflected light imaging section 113, the reflection spectrum generator 124 averages amounts of pieces of light received by light-receiving elements that are from among all of the light-receiving elements and situated at a center portion of all of the light-receiving elements, and sets an obtained average to be $A(t_0)$. The reflection spectrum generator 124 uses $A(t_0)$ for calculation of biological information. Here, the analysis processor 125 stores therein the amounts $A_{all}(t_0)$ of pieces of light received by all of the light-receiving elements.

When a next frame $t_1$ is reached after the elapse of a certain period of time, a mutual relationship between $A_{all}(t_1)$ and $A_{all}(t_0)$ is calculated to calculate an amount of shift $(x,y)$ of the entirety of a captured image. When the analysis processor 125 extracts $A(t)$ from $A_{all}(t)$, the analysis processor 125 performs an update by setting, to be a new center of the captured image, a position obtained by subtracting the calculated amount of shift $(x,y)$ from a position of the center of the captured image. It is assumed, in the examples illustrated in Figs. 7 and 8, that the center of a captured image at the time $t_0$ is represented by a point $P_0$ illustrated in Fig. 7, and the center of the captured image at the time $t_1$ is represented by a point $P_1$ illustrated in Fig. 8. Thereafter, the analysis processor 125 updates the center of the captured image over time to deal with an offset of a head-mounted display.

[Another Configuration of Biological Information Measuring Apparatus]

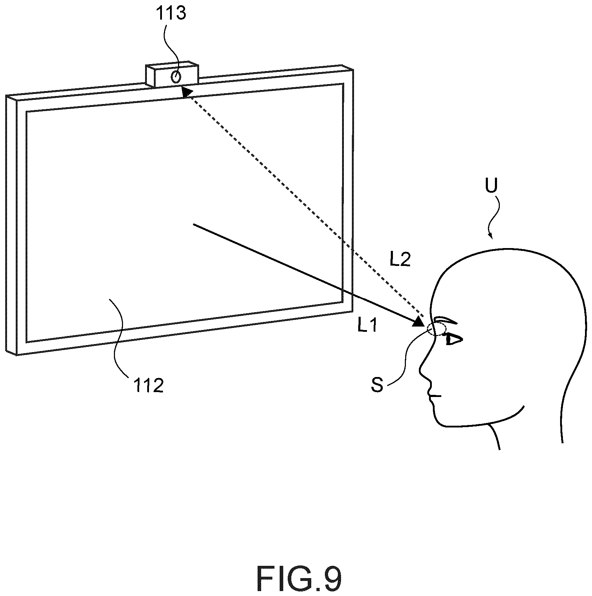

The example in which the biological information measuring apparatus 100 is a head-mounted display has been described above. However, the biological information measuring apparatus 100 may also have another configuration. Fig. 9 schematically illustrates the biological information measuring apparatus 100 having another configuration. As illustrated in the figure, the display section 112 and the reflected light imaging section 113 are each spaced from the user U. The display section 112 is a display apparatus that is not a head-mounted display, and is, for example, a TV apparatus or a screen in a movie theater. The reflected light imaging section 113 is an imaging device that is arranged to face the user U and that images the user U.

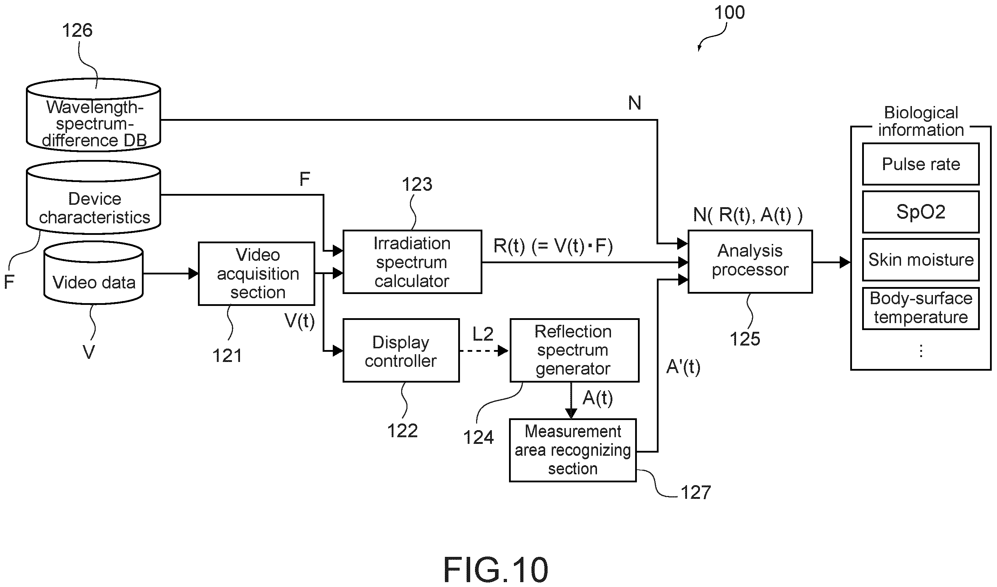

Fig. 10 is a block diagram illustrating a functional configuration of the biological information measuring apparatus 100 having the other configuration. As illustrated in the figure, the biological information measuring apparatus 100 may further include a measurement area recognizing section 127. The measurement area recognizing section 127 performs face recognition processing or facial parts recognizing processing with respect to an image obtained by imaging being performed by the reflected light imaging section 113, and recognizes the measurement area S. Further, the measurement area recognizing section 127 extracts a reflection spectrum $A'(t)$ in the measurement area S from the reflection spectrums $A(t)$ generated by the reflection spectrum generator 124, and supplies the extracted reflection spectrum $A'(t)$ to the analysis processor 125. The analysis processor 125 may apply the irradiation spectrum $R(t)$ and the reflection spectrum $A'(t)$ to the biological information calculating model N, and may calculate biological information from $N(R(t),A'(t))$.

When there is a plurality of users U in a field of view of the reflected light imaging section 113, the measurement area recognizing section 127 can recognize the measurement area S for each of the plurality of users U. In this case, the biological information measuring apparatus 100 can measure pieces of biological information regarding the plurality of users U at the same time. The measurement area S is not limited to the facial surface of the user U, and it is sufficient if the measurement area S is any area on a body surface of the user U such as an earlobe or a hand of which an image can be captured.

Note that there is a need to eliminate an effect of external light when there is the external light as in a normal room, although there is no need to do so when it is dark with no external light in a viewing environment of the user U as in a movie theater. Specifically, the measurement area recognizing section 127 calculates a distance from the user U to the reflected light imaging section 113, and specifies an illumination condition for a position of the user U. The analysis processor 125 determines whether the specified illumination condition is similar to an illumination condition upon constructing the biological information calculating model N. The analysis processor 125 may calculate biological information using the reflection spectrum $A'(t)$ when it has been determined that the two illumination conditions are similar to each other, and the analysis processor 125 may correct the reflection spectrum $A'(t)$ according to the difference and use the corrected reflection spectrum for calculation of the biological information when it has been determined that the two illumination conditions are different from each other.

(Second Embodiment)

A biological information detecting apparatus according to a second embodiment of the present technology is described.

[Configuration of Biological Information Detecting Apparatus]

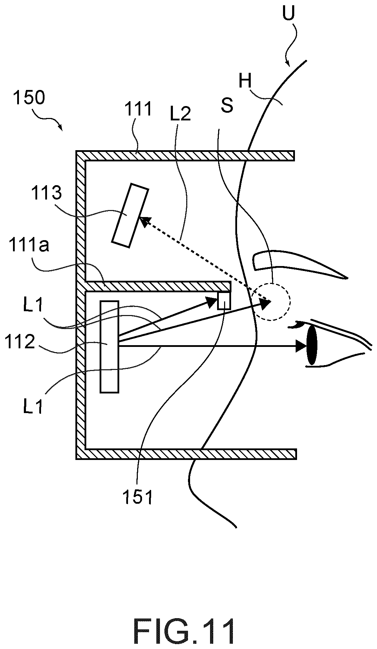

Fig. 11 schematically illustrates a biological information measuring apparatus 150 according to the present embodiment. As illustrated in the figure, the biological information measuring apparatus 150 is a head-mounted display worn on the head H of the user U, and includes the housing 111, the display section 112, the reflected light imaging section 113, and an irradiation light imaging section 151. Note that the structural elements other than the irradiation light imaging section 151 are similar to those of the biological information measuring apparatus 100 according to the first embodiment. Thus, descriptions thereof are omitted.

The irradiation light imaging section 151 images the irradiation light L1 to generate irradiation-light-reception data that is data of reception of the irradiation light L1. As illustrated in Fig. 11, the irradiation light imaging section 151 is arranged near the facial surface of the user U and faces the display section 112. As described above, emitted light emitted from the display section 112 is affected by, for example, the display characteristics of the display section 112, and is irradiated onto the facial surface of the user U in the form of the irradiation light L1. The irradiation light imaging section 151 images this irradiation light L1 to generate irradiation-light-reception data.

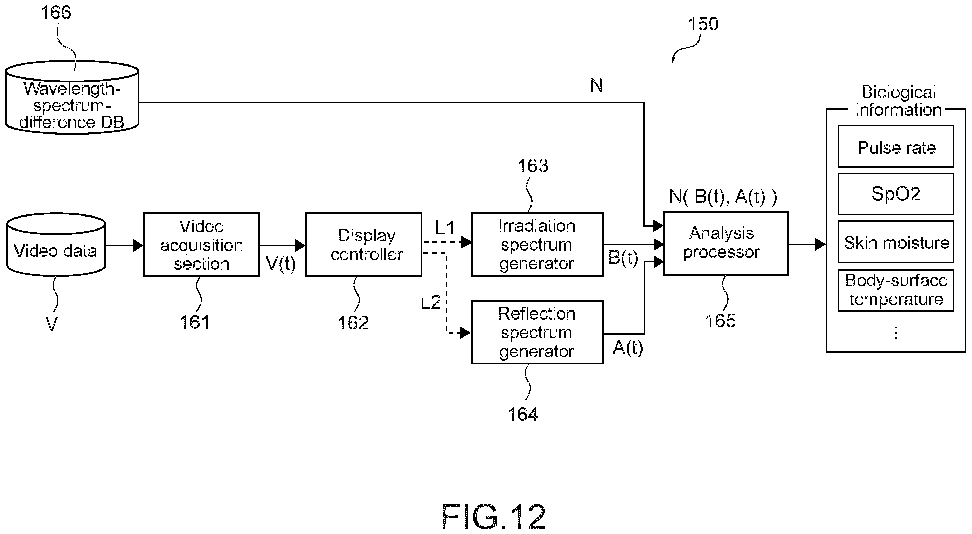

Fig. 12 is a block diagram illustrating a functional configuration of the biological information measuring apparatus 150 according to the present embodiment. As illustrated in the figure, the biological information measuring apparatus 150 includes a video acquisition section 161, a display controller 162, an irradiation spectrum generator 163, a reflection spectrum generator 164, and an analysis processor 165. The functional configuration is implemented by software and hardware working cooperatively. The functional configuration may be included in the housing 111 or in an apparatus external to the housing 111.

The video acquisition section 161 acquires video data V, and generates video data V(t) making up a display video displayed on the display section 112, as in the case of the first embodiment. The display controller 162 controls the display section 112 (refer to Fig. 11) to cause video data V(t) supplied by the video acquisition section 161 to be displayed on the display section 112. Accordingly, the irradiation light L1 making up a display video is irradiated onto a facial surface of a user.

The irradiation spectrum generator 163 acquires irradiation-light-reception data from the irradiation light imaging section 151, and generates an irradiation spectrum B(t) from the irradiation-light-reception data. The irradiation spectrum B(t) is a wavelength spectrum of the irradiation light L1, and is represented as indicated below.

$$B(t)=[b_1(t),b_2(t),\ \dots\ b_Q(t)] \qquad (Formula\ 6)$$

In (Formula 6), $b_i$ represents an amount of light output by a light-receiving element of a channel i of the irradiation light imaging section 151. Further, Q represents the number of channels of the irradiation light imaging section 151. The irradiation spectrum generator 163 may average amounts of pieces of light respectively output by all of the light-receiving elements of the irradiation light imaging section 151 to obtain a scalar, or may separately process amounts of pieces of light respectively output by the light-receiving elements. Further, the irradiation spectrum generator 163 may average to process amounts of pieces of light respectively output by a plurality of light-receiving elements. The irradiation spectrum calculator 123 calculates the irradiation spectrum R(t) in the first embodiment, whereas the irradiation spectrum B(t) generated from irradiation-light-reception data is used instead of calculation of the irradiation spectrum R(t) in the present embodiment. The irradiation spectrum generator 163 supplies the generated reflection spectrum B(t) to the analysis processor 165.

The reflection spectrum generator 164 acquires reflected-light-reception data from the reflected light imaging section 113, and generates a reflection spectrum A(t) from the reflected-light-reception data. The reflection spectrum A(t) is a wavelength spectrum of the reflected light L2, as in the case of the first embodiment. The reflection spectrum generator 164 supplies the generated reflection spectrum A(t) to the analysis processor 165.

The analysis processor 165 calculates biological information regarding a user on the basis of the irradiation spectrum B(t) and the reflection spectrum A(t). As in the case of the first embodiment, there is a difference (refer to Fig. 5) between the irradiation spectrum B(t) and the reflection spectrum A(t). Biological information in the measurement area S is reflected in characteristics of the difference, that is, in a wavelength band in which there is the difference and in an amount of change in intensity in which there is the difference. Thus, the analysis processor 165 can calculate biological information regarding a user on the basis of the irradiation spectrum B(t) and the reflection spectrum A(t).

Specifically, the analysis processor 165 can acquire a biological information calculating model N from the wavelength-spectrum-difference database 166. The biological information calculating model N is obtained by performing modeling on an irradiation spectrum B(t), a reflection spectrum A(t), and biological information for each type of biological information. As represented by (Formula 7) indicated below, the analysis processor 165 can calculate biological information (index) by applying the irradiation spectrum B(t) and the reflection spectrum A(t) to the biological information calculating model N.

$$N(B(t),A(t))=index \qquad (Formula\ 7)$$

As in the case of the first embodiment, examples of the biological information that can be calculated using the biological information calculating model N include a pulse rate, arterial oxygen saturation (SpO2), a skin-moisture amount, a body-surface temperature, a breathing rate, a blood pressure, a vein pattern, and an iris pattern. The analysis processor 165 selects the biological information calculating model N according to the type of biological information to be calculated, and can use the selected biological information calculating model N to calculate biological information.

The biological information calculating model N may be a predictive model generated by a machine learning approach in which the irradiation spectrum B(t) and the reflection spectrum A(t) are used as input and biological information measured using, for example, a wearable device is used as training data. Further, the biological information calculating model N is not limited to a model generated by machine learning. A specific example of biological information calculated by the analysis processor 165 using the biological information calculating model N is similar to the specific example in the first embodiment, and thus it is sufficient if the irradiation spectrum R(t) is replaced with the irradiation spectrum R(t) in the description of the first embodiment. The analysis processor 165 can also perform biometric authentication on the basis of a measured vein pattern or iris pattern.

[Operation of Biological Information Detecting Apparatus]

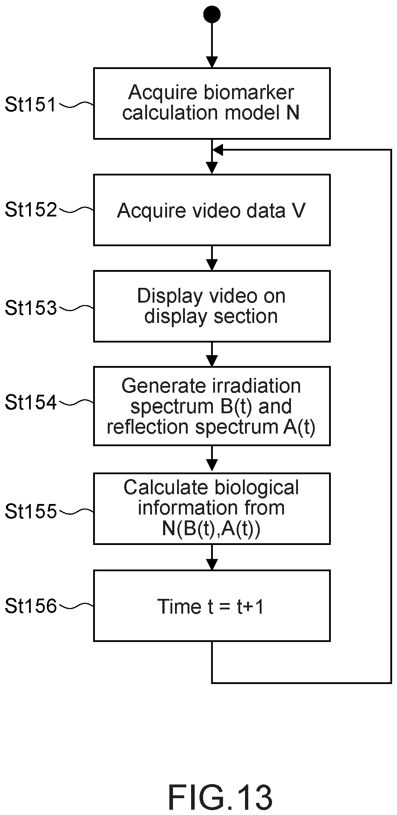

An operation of the biological information measuring apparatus 150 is described. Fig. 13 is a flowchart illustrating the operation of the biological information measuring apparatus 150.

When the biological information measuring apparatus 150 starts operating, the analysis processor 165 acquires a biological information calculation model N for calculation-target biological information from the wavelength-spectrum-difference database 166 (St151). Subsequently, the video acquisition section 161 acquires video data V (St152), and the display controller 162 supplies video data V(t) to the display section 112 to cause a display video to be displayed on the display section 112 (St153).

Subsequently, the irradiation spectrum generator 163 acquires irradiation-light-reception data from the irradiation light imaging section 151, and generates a reflection spectrum B(t) from the irradiation-light-reception data (St154). Further, the reflection spectrum generator 164 acquires reflected-light-reception data from the reflected light imaging section 113, and generates a reflection spectrum A(t) from the reflected-light-reception data (St154).

Subsequently, the analysis processor 165 applies the irradiation spectrum B(t) and the reflection spectrum A(t) to the biological information calculating model N, and calculates biological information from N(B(t),A(t)) (St155). Subsequently, the analysis processor 165 changes the time from a time t to a time t+1 (St156), and repeatedly performs the processes of and after the acquisition of the biological information calculating model N (St151) again. Note that the time t corresponds to a frame of the video data V(t), and the time t+1 means addition of one frame.

The biological information measuring apparatus 150 measures biological information as described above. Note that the irradiation light imaging data and the reflected light imaging data may be stored upon the generation of the irradiation spectrum B(t) and the reflection spectrum A(t) (St154), and a processing apparatus such as a server with a high computational performance may calculate N(B(t),A(t)) using the irradiation light imaging data and the reflected light imaging data afterward together with the video data V(t).

[Effects Provided by Biological Information Detecting Apparatus]

As in the case of the biological information measuring apparatus 100 according to the first embodiment, the biological information measuring apparatus 150 can measure biological information on the basis of a difference between light irradiated onto a facial surface of a user and light reflected off the facial surface of the user. This results in a reduction in burdens imposed on the user upon measuring the biological information. Compared with the biological information measuring apparatus 100, there is a need to provide the irradiation light imaging section 151 to the biological information measuring apparatus 150, whereas neither premeasurement of the device characteristics F nor processing of calculating the irradiation spectrum R(t) are necessary.

[Others]

Using an approach similar to the approach applied in the first embodiment, the biological information measuring apparatus 150 can deal with an offset of a head-mounted display that is the biological information measuring apparatus 150 (refer to Figs. 7 and 8). Further, the biological information measuring apparatus 150 may also be an apparatus other than a head-mounted display, as in the case of the first embodiment (refer to Figs. 9 and 10).

[Hardware Configuration of Biological Information Measuring Apparatus]

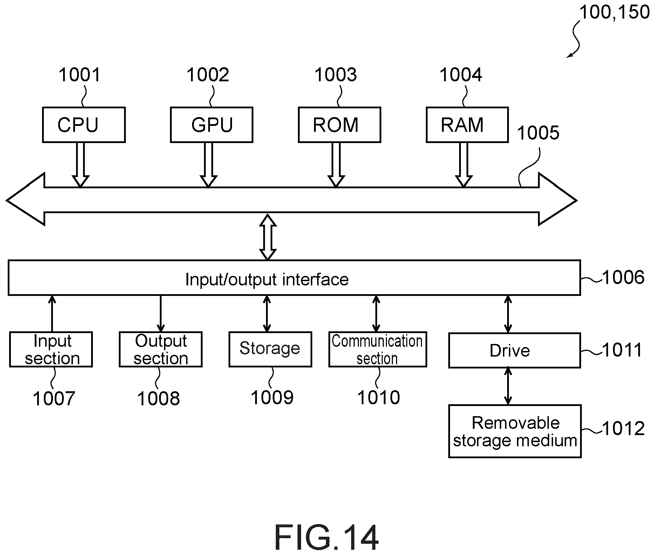

A hardware configuration that makes it possible to implement a functional configuration of each of the biological information measuring apparatus 100 according to the first embodiment and the biological information measuring apparatus 150 according to the second embodiment is described. Fig. 14 schematically illustrates the hardware configuration.

As illustrated in the figure, each of the biological information measuring apparatus 100 and the biological information measuring apparatus 150 includes a central processing unit (CPU) 1001 and a graphics processing unit (GPU) 1002. An input/output interface 1006 is connected to the CPU 1001 and the GPU 1002 via a bus 1005. A read only memory (ROM) 1003 and a random access memory (RAM) 1004 are connected to the bus 1005.

An input section 1007, an output section 1008, a storage 1009, and a communication section 1010 are connected to the input/output interface 1006. The input section 1007 includes input devices such as a keyboard and a mouse that are used by a user to input an operation command. The output section 1008 outputs a processing operation screen and an image of a processing result to a display device. The storage 1009 includes, for example, a hard disk drive that stores therein a program and various data. The communication section 1010 includes, for example, a local area network (LAN) adapter, and performs communication processing through a network as represented by the Internet. Further, a drive 1011 is connected to the input/output interface 1006. The drive 1011 reads data from and writes data into a removable storage medium 1012 such as a magnetic disk, an optical disk, a magneto-optical disk, or a semiconductor memory.

The CPU 1001 performs various processes in accordance with a program stored in the ROM 1003, or in accordance with a program that is read from the removable storage medium 1012 such as a magnetic disk, an optical disk, a magneto-optical disk, or a semiconductor memory to be installed on the storage 1009, and is loaded into the RAM 1004 from the storage 1009. Data necessary for the CPU 1001 to perform various processes is also stored in the RAM 1004 as necessary. The GPU 1002 performs calculation processing necessary to draw an image under the control of the CPU 1001.

In the biological information measuring apparatus 100 and biological information measuring apparatus 150 each having the configuration described above, the series of processes described above is performed by the CPU 1001 loading, for example, a program stored in the storage 1009 into the RAM 1004 and executing the program via the input/output interface 1006 and the bus 1005.

For example, the program executed by each of the biological information measuring apparatus 100 and biological information measuring apparatus 150 can be provided by being recorded in the removable storage medium 1012 serving as, for example, a package medium. Further, the program can be provided via a wired or wireless transmission medium such as a local area network, the Internet, or digital satellite broadcasting.

In each of the biological information measuring apparatus 100 and biological information measuring apparatus 150, the program can be installed on the storage 1009 via the input/output interface 1006 by the removable storage medium 1012 being mounted on the drive 1011. Further, the program can be received by the communication section 1010 via the wired or wireless transmission medium to be installed on the storage 1009. Moreover, the program can be installed in advance on the ROM 1003 or the storage 1009.

Note that the program executed by each of the biological information measuring apparatus 100 and biological information measuring apparatus 150 may be a program in which processes are chronologically performed in the order of the description in the present disclosure, or may be a program in which processes are performed in parallel or a process is performed at a necessary timing such as a timing of calling. Further, all of the hardware configuration of each of the biological information measuring apparatus 100 and biological information measuring apparatus 150 does not have to be included in a single apparatus, and each of the biological information measuring apparatus 100 and biological information measuring apparatus 150 may include a plurality of apparatuses. Further, a portion of or all of the hardware configuration of each of the biological information measuring apparatus 100 and biological information measuring apparatus 150 may be included in a plurality of apparatuses connected to each other via a network.

(Regarding Present Disclosure)

The effects described in the present disclosure are not limitative but are merely illustrative, and other effects may be provided. The above-described description of the plurality of effects does not necessarily mean that the plurality of effects is provided at the same time. The above-described description means that at least one of the effects described above is provided depending on, for example, a condition. There is a possibility that an effect that is not described in the present disclosure will be provided. Further, at least two of the features described in the present disclosure can also be used in combination.

Note that the present technology may also take the following configurations.

(1) A biological information measuring apparatus, including:

a reflection spectrum generator that generates a reflection spectrum that is a wavelength spectrum of reflected light obtained by irradiation light irradiated onto a measurement area being reflected off the measurement area, the irradiation light making up a display video, the measurement area being a specific area on a body of a user; and

an analysis processor that calculates biological information regarding the user on the basis of an irradiation spectrum and the reflection spectrum, the irradiation spectrum being a wavelength spectrum of the irradiation light.

(2) The biological information measuring apparatus according to (1), in which
the reflection spectrum generator generates the reflection spectrum from reflected-light-reception data that is data generated by the reflected light being imaged.
(3) The biological information measuring apparatus according to (1) or (2), further including

an irradiation spectrum calculator that calculates the irradiation spectrum on the basis of video data of the display video and on the basis of device characteristics of the biological information measuring apparatus, in which
the spectrum acquisition section acquires the irradiation spectrum from the irradiation spectrum calculator.

(4) The biological information measuring apparatus according to (1) or (2), further including

an irradiation spectrum generator that generates the irradiation spectrum from irradiation-light-reception data that is data generated by the irradiation light being imaged, in which
the spectrum acquisition section acquires the irradiation spectrum from the irradiation spectrum calculator.

(5) The biological information measuring apparatus according to (1), further including:

a display section that displays thereon the display video; and
a reflected light imaging section that images the reflected light to generate reflected-light-reception data.

(6) The biological information measuring apparatus according to (5), further including
an irradiation light imaging section that images the irradiation light to generate irradiation-light-reception data.
(7) The biological information measuring apparatus according to (5), in which
the biological information measuring apparatus is a head-mounted display that is worn on a head of the user and of which the display section is arranged in front of eyes of the user.
(8) The biological information measuring apparatus according to (7), in which
the reflected light imaging section is arranged to face the measurement area.
(9) The biological information measuring apparatus according to (8), further including

an irradiation light imaging section that images the irradiation light to generate irradiation-light-reception data, in which
the irradiation light imaging section is arranged to face the display section.

(10) The biological information measuring apparatus according to (5), in which

the display section and the reflected light imaging section are each spaced from the user, and
the biological information measuring apparatus further includes a measurement area recognizing section that recognizes the measurement area on the basis of an image obtained by imaging being performed by the reflected light imaging section.

(11) The biological information measuring apparatus according to any one of (1) to (10), in which
the analysis processor determines the biological information regarding the user using a biological information calculating model obtained by performing modeling on the irradiation spectrum, the reflection spectrum, and the biological information for each type of biological information.
(12) The biological information measuring apparatus according to (11), in which
the biological information calculating model is a predictive model that is generated by machine learning.
(13) The biological information measuring apparatus according to any one of (1) to (12), in which
the biological information is a pulse rate, arterial oxygen saturation, a skin-moisture amount, a body-surface temperature, a breathing rate, or a blood pressure.
(14) The biological information measuring apparatus according to any one of (1) to (12), in which

the biological information is a vein pattern or an iris pattern, and
the analysis processor performs biometric authentication using the biological information.

**EP 4 520 259 A1**

(15) A biological information measuring program that causes an information processing apparatus to operate as a reflection spectrum generator and an analysis processor,

the reflection spectrum generator generating a reflection spectrum that is a wavelength spectrum of reflected light obtained by irradiation light irradiated onto a measurement area being reflected off the measurement area, the irradiation light making up a display video, the measurement area being a specific area on a body of a user, the analysis processor calculating biological information regarding the user on the basis of an irradiation spectrum and the reflection spectrum, the irradiation spectrum being a wavelength spectrum of the irradiation light.

(16) A biological information measuring method, including:

generating a reflection spectrum that is a wavelength spectrum of reflected light obtained by irradiation light irradiated onto a measurement area being reflected off the measurement area, the irradiation light making up a display video, the measurement area being a specific area on a body of a user; and
calculating biological information regarding the user on the basis of an irradiation spectrum and the reflection spectrum, the irradiation spectrum being a wavelength spectrum of the irradiation light.

Reference Signs List

100, 150 biological information measuring apparatus
111 housing
112 display section
113 reflected light imaging section
121, 161 video acquisition section
122, 162 display controller
123 irradiation spectrum calculator
124, 164 reflection spectrum generator
125, 165 analysis processor
126, 166 wavelength-spectrum-difference database
127 measurement area recognizing section
151 irradiation light imaging section
163 irradiation spectrum generator

**Claims**

1.  A biological information measuring apparatus, comprising:

a reflection spectrum generator that generates a reflection spectrum that is a wavelength spectrum of reflected light obtained by irradiation light irradiated onto a measurement area being reflected off the measurement area, the irradiation light making up a display video, the measurement area being a specific area on a body of a user; and
an analysis processor that calculates biological information regarding the user on a basis of an irradiation spectrum and the reflection spectrum, the irradiation spectrum being a wavelength spectrum of the irradiation light.

2.  The biological information measuring apparatus according to claim 1, wherein
the reflection spectrum generator generates the reflection spectrum from reflected-light-reception data that is data generated by the reflected light being imaged.

3.  The biological information measuring apparatus according to claim 1, further comprising

an irradiation spectrum calculator that calculates the irradiation spectrum on a basis of video data of the display video and on a basis of device characteristics of the biological information measuring apparatus, wherein
the spectrum acquisition section acquires the irradiation spectrum from the irradiation spectrum calculator.

4. The biological information measuring apparatus according to claim 1, further comprising

an irradiation spectrum generator that generates the irradiation spectrum from irradiation-light-reception data that is data generated by the irradiation light being imaged, wherein
the spectrum acquisition section acquires the irradiation spectrum from the irradiation spectrum calculator.

5. The biological information measuring apparatus according to claim 1, further comprising:

a display section that displays thereon the display video; and
a reflected light imaging section that images the reflected light to generate reflected-light-reception data.

6. The biological information measuring apparatus according to claim 5, further comprising
an irradiation light imaging section that images the irradiation light to generate irradiation-light-reception data.

7. The biological information measuring apparatus according to claim 5, wherein
the biological information measuring apparatus is a head-mounted display that is worn on a head of the user and of which the display section is arranged in front of eyes of the user.

8. The biological information measuring apparatus according to claim 7, wherein
the reflected light imaging section is arranged to face the measurement area.

9. The biological information measuring apparatus according to claim 8, further comprising

an irradiation light imaging section that images the irradiation light to generate irradiation-light-reception data, wherein
the irradiation light imaging section is arranged to face the display section.

10. The biological information measuring apparatus according to claim 5, wherein

the display section and the reflected light imaging section are each spaced from the user, and
the biological information measuring apparatus further comprises a measurement area recognizing section that recognizes the measurement area on a basis of an image obtained by imaging being performed by the reflected light imaging section.

11. The biological information measuring apparatus according to claim 1, wherein
the analysis processor determines the biological information regarding the user using a biological information calculating model obtained by performing modeling on the irradiation spectrum, the reflection spectrum, and the biological information for each type of biological information.

12. The biological information measuring apparatus according to claim 11, wherein
the biological information calculating model is a predictive model that is generated by machine learning.

13. The biological information measuring apparatus according to claim 1, wherein
the biological information is a pulse rate, arterial oxygen saturation, a skin-moisture amount, a body-surface temperature, a breathing rate, or a blood pressure.

14. The biological information measuring apparatus according to claim 1, wherein

the biological information is a vein pattern or an iris pattern, and
the analysis processor performs biometric authentication using the biological information.

15. A biological information measuring program that causes an information processing apparatus to operate as a reflection spectrum generator and an analysis processor,

the reflection spectrum generator generating a reflection spectrum that is a wavelength spectrum of reflected light obtained by irradiation light irradiated onto a measurement area being reflected off the measurement area, the irradiation light making up a display video, the measurement area being a specific area on a body of a user, the analysis processor calculating biological information regarding the user on a basis of an irradiation spectrum and the reflection spectrum, the irradiation spectrum being a wavelength spectrum of the irradiation light.

**16.** A biological information measuring method, comprising:

generating a reflection spectrum that is a wavelength spectrum of reflected light obtained by irradiation light irradiated onto a measurement area being reflected off the measurement area, the irradiation light making up a display video, the measurement area being a specific area on a body of a user; and

calculating biological information regarding the user on a basis of an irradiation spectrum and the reflection spectrum, the irradiation spectrum being a wavelength spectrum of the irradiation light.

FIG.1

FIG.2

FIG.3

FIG.4

FIG.5

St101 — Acquire biomarker calculation model N

St102 — Acquire video data V

St103 — Display video on display section

St104 — Calculate irradiation spectrum R(t)

St105 — Generate reflection spectrum A(t)

St106 — Calculate biological information from N(R(t),A(t))

St107 — Time t = t+1

FIG.6

FIG.7

FIG.8

FIG.9

FIG.10

FIG.11

FIG.12

EP 4 520 259 A1

St151 — Acquire biomarker calculation model N

St152 — Acquire video data V

St153 — Display video on display section

St154 — Generate irradiation spectrum B(t) and reflection spectrum A(t)

St155 — Calculate biological information from N(B(t),A(t))

St156 — Time t = t+1

FIG.13

100,150

| 1001 | 1002 | 1003 | 1004 | |
|------|------|------|------|---|
| CPU | GPU | ROM | RAM | |

1005

Input/output interface ~1006

| Input section | Output section | Storage | Communication section | | Drive | ~1011 |
|---|---|---|---|---|---|---|

1007　　1008　　1009　　1010

Removable storage medium ~1012

FIG.14

## INTERNATIONAL SEARCH REPORT

| International application No. |
| --- |
| **PCT/JP2023/014497** |

**A. CLASSIFICATION OF SUBJECT MATTER**

*A61B 5/02*(2006.01)i; *A61B 5/00*(2006.01)i; *A61B 5/01*(2006.01)i; *A61B 5/021*(2006.01)i; *A61B 5/0245*(2006.01)i; *A61B 5/08*(2006.01)i; *A61B 5/1171*(2016.01)i; *A61B 5/1455*(2006.01)i; *G02B 27/02*(2006.01)i; *H04N 5/64*(2006.01)i
FI: A61B5/02 B; A61B5/0245 200; A61B5/08; A61B5/1171 100; A61B5/1171 300; A61B5/1455; H04N5/64 511A; A61B5/01 350; A61B5/00 M; A61B5/021; G02B27/02 Z

According to International Patent Classification (IPC) or to both national classification and IPC

**B. FIELDS SEARCHED**

Minimum documentation searched (classification system followed by classification symbols)

A61B5/00-5/398; A61B3/113; G06V40/00-40/70; G06F21/32

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2021-18729 A (SONY INTERACTIVE ENTERTAINMENT LLC) 15 February 2021 (2021-02-15) paragraphs [0029]-[0039], [0056] | 1-2, 5, 10, 14-16 |
| Y | | 1-16 |
| Y | JP 2021-532891 A (DEUTSCHES KREBSFORSCHUNGSZENTRUM STIFTUNG DES OEFFENTLICHEN RECHTS) 02 December 2021 (2021-12-02) paragraphs [0004], [0011], [0013], [0032], [0053] | 1-16 |
| A | US 5583795 A (THE UNITED STATES OF AMERICA AS REPRESENTED BY THE SECRETARY OF THE ARMY) 10 December 1996 (1996-12-10) column 6, lines 39-60 | 1-16 |
| A | US 6120461 A (THE UNITED STATES OF AMERICA AS REPRESENTED BY THE SECRETARY OF THE ARMY) 19 September 2000 (2000-09-19) column 7, line 1 to column 8, line 13 | 1-16 |

☑ Further documents are listed in the continuation of Box C. ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **25 April 2023** | **20 June 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2023/014497**

| C. | DOCUMENTS CONSIDERED TO BE RELEVANT | | |
|---|---|---|---|
| Category* | Citation of document, with indication, where appropriate, of the relevant passages | | Relevant to claim No. |
| A | US 2017/0231490 A1 (AUTONOMIX MEDICAL, INC.) 17 August 2017 (2017-08-17) paragraph [0293] | | 1-16 |
| A | WO 2021/261100 A1 (SONY GROUP CORP) 30 December 2021 (2021-12-30) paragraphs [0002]-[0013] | | 1-16 |

Form PCT/ISA/210 (second sheet) (January 2015)

**INTERNATIONAL SEARCH REPORT**
Information on patent family members

International application No.

**PCT/JP2023/014497**

| Patent document cited in search report | | | Publication date (day/month/year) | Patent family member(s) | Publication date (day/month/year) |
|---|---|---|---|---|---|
| JP | 2021-18729 | A | 15 February 2021 | (Family: none) | |
| JP | 2021-532891 | A | 02 December 2021 | US 2022/0008157 A1<br>paragraphs [0004], [0012], [0014]-[0017], [0037], [0058]<br>WO 2020/025684 A1<br>EP 3829416 A1 | |
| US | 5583795 | A | 10 December 1996 | (Family: none) | |
| US | 6120461 | A | 19 September 2000 | (Family: none) | |
| US | 2017/0231490 | A1 | 17 August 2017 | WO 2016/025323 A1<br>EP 3177201 A1 | |
| WO | 2021/261100 | A1 | 30 December 2021 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021018729 A